# EUROPEAN PATENT SPECIFICATION

(11) **EP 2 088 916 B1**
(45) Date of publication and mention of the grant of the patent: **28.08.2013**
(21) Application number: 07862556.3
(22) Date of filing: 04.12.2007
(51) Int. Cl.: A61M 1/00, A61B 1/015, A61B 1/313

(54) **APPARATUS FOR AN ENDOSCOPE PUMP**
VORRICHTUNG FÜR EINE ENDOSKOPPUMPE
APPAREIL POUR UNE POMPE D'ENDOSCOPE

(30) Priority: 04.12.2006 US 868458 P; 03.12.2007 US 949250
(43) Date of publication of application: 19.08.2009
(73) Proprietor: Karl Storz Endovision, Inc., Charlton, MA 01507 (US)
(72) Inventor: ISAACSON, Keith B., Newton, MA 02458 (US)
(74) Representative: Witte, Weller & Partner
(86) International application number: PCT/US2007/024920
(87) International publication number: WO 2008/070123

(56) References cited:
- EP-A2- 0 529 902
- WO-A2-01/37922
- DE-U1- 9 410 530
- US-A- 3 020 017
- US-A- 5 810 770
- US-A1- 2002 177 803
- US-A1- 2006 106 285

## Description

The invention relates to a fluid pump system for an endoscopic surgical instrument, the system comprising a housing, a disposable inflow cartridge, detachably connected to the housing, a disposable outflow cartridge, detachably connected to the housing, and at least one fluid pump connected to the housing, wherein the at least one fluid pump is in communication with at least one of the disposable inflow cartridge and the disposable outflow cartridge.

A fluid pump system of this kind is known from US 2002/177803 A1.
Similar devices are known from WO 01/37922 A2, DE 94 10 53 U1 and EP 0 529 902 A2.

Devices that deliver irrigating fluid to an irrigation site are necessary in a variety of medical procedures, particularly procedures utilizing endoscopic surgical techniques. For example, laparoscopic, arthroscopic, and hysteroscopic procedures require sufficient volumes of irrigation fluid to be delivered to the surgical site in order to maintain tamponade, isolate bleeders, and to generally clear the surgical area. Laparoscopic procedures involve incisions to the abdominal cavity and include appendectomies, cholecystectcomy (incision of the gall bladder) and treatment of ectopic pregnancies. Hysteroscopic procedures involve inspection of the uterine cavity and include procedures that remove abnormal tissue from the uterus such as a biopsy or a myomectomy. Arthroscopic procedures are typically performed by an orthopedic surgeon and involve irrigation, distension and inspection of the joints such as at the knee, shoulder, elbow or ankle. Such arthroscopic procedures include synovectomy, meniscectomy, or repair of the anterior cruciate ligament.

During these and various other surgical procedures, it is also generally useful for the surgeon to keep tissue that surrounds the surgical site out of the way by injecting solutions such as saline, glycine, lactated Ringer's solution, or the like to the surgical site. This is particularly important during endoscopic surgery in order to provide enough room to accommodate the surgical tools and to allow for the performance of surgical tasks. Also, it is often desirable for a surgeon to have the ability to evacuate fluids or tissue from the surgical site during a surgical procedure. This includes, for example, removing tissue that has been resected during the procedure, as well as the removal of excess fluids.

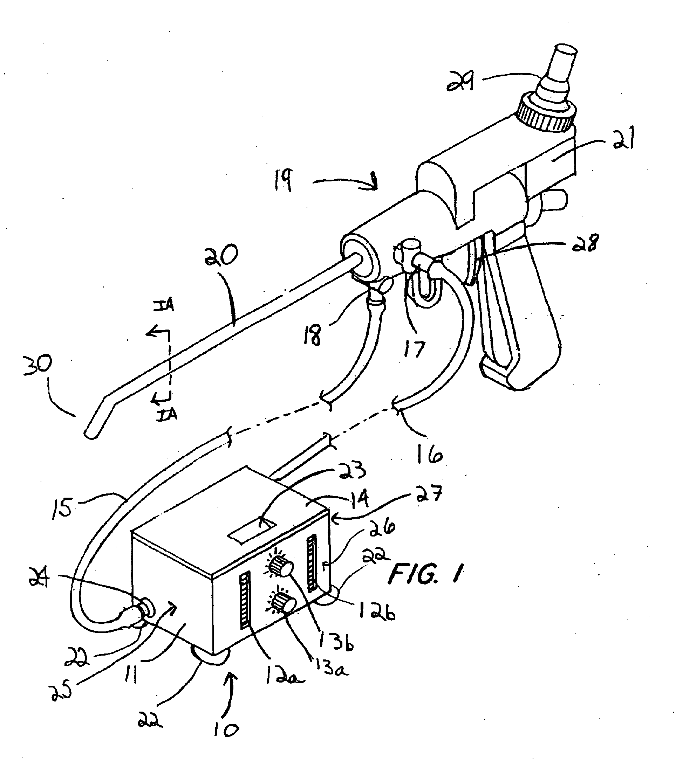

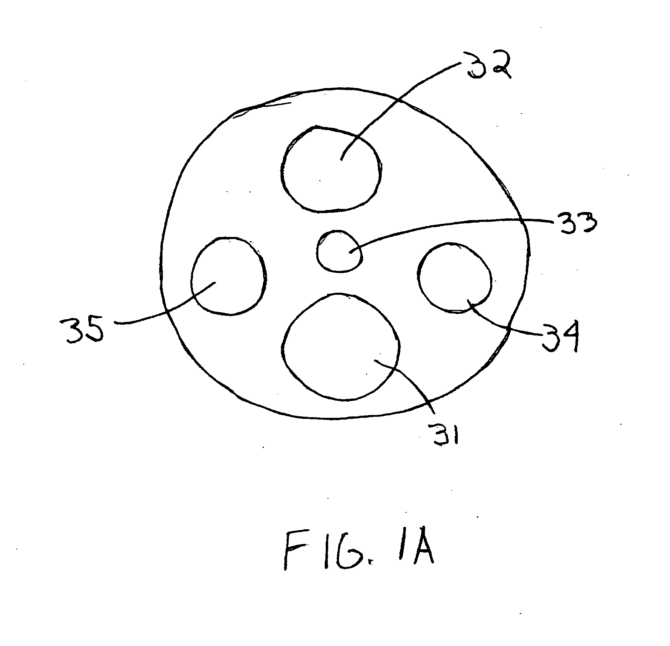

The delivery to and evacuation of fluids and/or tissue from a surgical site is often accomplished using a pump system in conjunction with a surgical tool of some kind. In many cases, an endoscopic surgical instrument will be adapted to deliver and evacuate fluids while also providing an image of the surgical site and the ability to operate a surgical tool of some kind at the surgical site. An example of an endoscopic surgical instrument is shown in Fig. 1. The instrument has a shaft 20 for insertion into a patient's body via a body cavity or an incision. Such an instrument has a plurality of channels running along the shaft. At least one of these channels guides a surgical tool, such as clippers or an electrode, and another of these channels contains means for providing an image of the surgical site to a video monitor or directly to the surgeon's eye. The instrument also has a channel out of which fluids flow to a surgical site and a channel through which fluids are removed from a surgical site. A pump system connects to instrument inlet and outlet ports 17 and 18 for the supply and removal of fluids and/or tissue.

A pump system for supplying and/or evacuating fluids from a surgical site must meet certain requirements in order to be useful and practical to surgeons and other health care providers, as well as safe for surgical patients. First, the sterility of all of the components involved in the surgery is of paramount importance. While sterility of surgical components and devices is of the utmost importance, it can also be costly. Expensive and complicated surgical equipment that must be sterilized after every use will quickly increase the health care provider's cost of services. One important way of reducing the costs associated with maintaining sterility is to provide many surgical devices and components as single-use, disposable units.

Second, a pump system must be simple and easy to use by the surgeon or operator. While a surgery is underway, it is imperative to minimize the amount of distraction to the surgeon and his or her staff caused by complicated equipment. The system should also be unobtrusive and space-efficient. Further, the pump system should be stable and provide other safeguards against the spillage of fluids that may jeopardize the sterility of the environment or cause other safety hazards.

Many different pump systems have been developed for providing the fluid to the endoscopic surgical instrument and for providing means to evacuate fluid from the surgical site. However, the pump systems of the prior art suffer from significant drawbacks and do not adequately address the foregoing needs.

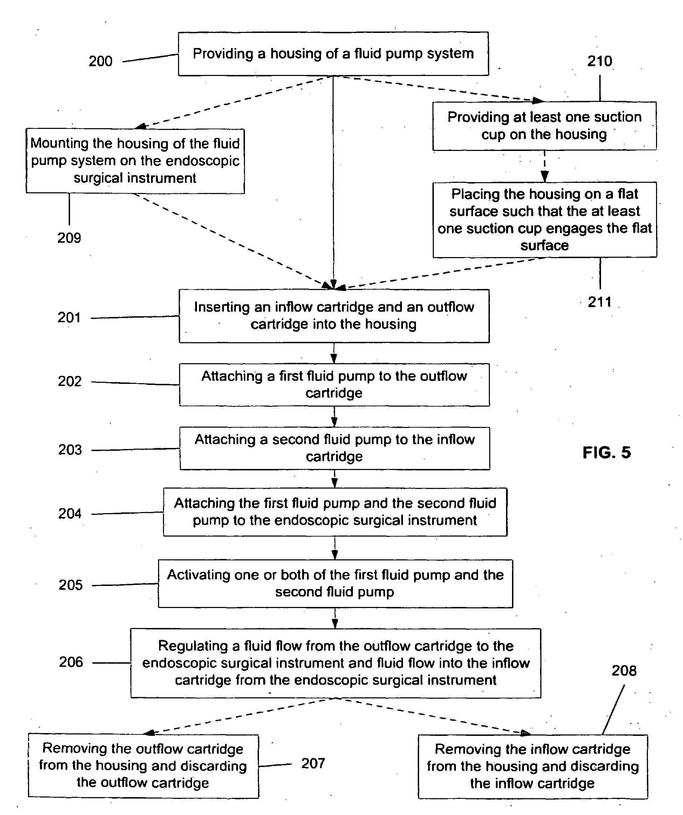

For example, U.S. Patent Appl. Pub. No 2006/0106285 of Boulais et al. discloses a fluid delivery system for use with an endoscope. The system includes multiple fluid sources that are disposable. One or more pump units are used to move fluid from the fluid sources through the endoscope to a surgical site and from the surgical site through the endoscope to a collection jar. However, as shown in Figures 5 and 8 of the Boulais et al. application, the components of the fluid delivery system are separately mounted on a large console. The large size and complexity of the system described in the Boulais et al. application make it unsuitable for many smaller outpatient facilities or physician's offices which may have confined space.

U.S. Patent No. 6,840,902 to Sano et al. discloses an endoscope that has a tank for storing liquid and a pump for pumping liquid to the distal end of the endoscope. The tank and pump mount directly on the operational portion (the handle) of the endoscope. A battery is provided for powering the pump. Sano et al. also discloses that the absorption of fluids may be accomplished via a separate absorption system.

While the pump system described by Sano et al. may be small and unobtrusive, it also suffers from obvious drawbacks. For example, the separate nature of the systems for fluid supply and fluid absorption increase the complexity of use and maintenance of the system. Second, the reusable nature of the fluid supply system means that sterilization costs are a factor. This is exacerbated by the small size of the fluid tank, since it is likely that multiple units would be necessary for complicated surgeries and every fluid tank would need to be sterilized after use. Therefore, the costs of using this system would be high.

U.S. Patent 5,395,312 to Desai discloses a handheld surgical instrument with a front end to which a surgical tool is mounted. The instrument includes a removable and disposable valve cartridge that has an irrigation tube and an evacuation tube. Desai also discloses the use of flexible chambers that are manipulated in order to pump fluid from a fluid supply source to the surgical site in a patient. Drive means act on the chambers to move the fluid from the chamber along a conduit to the patient's body. The fluids are supplied by a separate source via an inlet port. Evacuation of fluids from the patient is accomplished by a separate source of suction.

The system disclosed in Desai also suffers from significant drawbacks similar to those described above. The irrigation system and the suction system are separate, which adds to the complexity of operation and maintenance of the system. The flexible chambers used to pump the fluid are also separate from the other components of the system and must be removed from the instrument for sterilization. Overall, the system described by Desai is complicated and expensive to maintain and operate.

What is desired, therefore, is a fluid pump system for delivering and/or evacuating fluids or other matter to or from a surgical site that is simple and inexpensive to use and maintain, that minimizes the costs of maintaining sterility, that does not impede a surgeon by its size or weight during a surgical procedure, and that is simple and inexpensive to manufacture.

### Summary Of The Invention

It is an object of the present invention to provide a fluid pump system that is simple and inexpensive to use and maintain.

It is a further object of the present invention to provide a fluid pump system that minimizes the costs of maintaining sterility.

It is yet another object of the present invention to provide a fluid pump system that does not impede a surgeon by its size or weight during a surgical procedure.

It is still another object of the present invention to provide a fluid pump system that is simple and inexpensive to manufacture.

These objects are achieved by an endoscopic surgical instrument having a shaft for insertion into the body of a patient, and in that the housing is detachably mounted on the endoscopic surgical instrument.
The present invention is defined in the appended claims.

In some embodiments, an endoscopic surgical instrument is in fluid communication with at least one of the disposable inflow cartridge and the disposable outflow cartridge. In some embodiments, the housing includes at least one control device for controlling the at least one fluid pump, thereby regulating the flow of an irrigation fluid from the outflow cartridge. In some embodiments, the housing includes at least one control device for controlling the at least one fluid pump, thereby regulating the flow of an irrigation fluid into the inflow cartridge.

In some embodiments, the housing includes at least one suction device for securing the housing on a surface. In some embodiments, the at least one fluid pump is detachably connected to the housing and is disposable. In some embodiments, a first fluid pump is in fluid communication with the inflow cartridge and a second fluid pump is in fluid communication with the outflow cartridge. In some embodiments, the first fluid pump evacuates fluid from an inflow channel of an endoscopic surgical instrument and the second fluid pump supplies fluid to an outflow channel of an endoscopic surgical instrument. The housing is adapted to be detachably mounted on an endoscopic surgical instrument.

According to a exemplary embodiment of the present invention, a fluid pump system for an endoscopic surgical instrument, is provided, the system comprising: a housing, adapted to be detachably mounted on an endoscopic surgical instrument; a disposable inflow cartridge, detachably connected to the housing; a disposable outflow cartridge, detachably connected to the housing; a first fluid pump connected to the housing and in fluid communication with the disposable inflow cartridge; and a second fluid pump connected to the housing and in fluid communication with the disposable outflow cartridge. The first fluid pump is in fluid communication with an inflow channel of the endoscopic surgical instrument and the second fluid pump is in fluid communication with an outflow channel of the endoscopic surgical instrument.

In some embodiments, the housing includes at least one control device for regulating flow of a fluid from the outflow cartridge and at least one control device for regulating flow of a fluid into the inflow cartridge. In some embodiments, the first fluid pump and the second fluid pump are detachably connected to the housing. In some embodiments, the fluid pump system further comprises at least one power supply for driving the first fluid pump and the second fluid pump. The housing includes recesses for detachably connecting suction cups thereto.

A method for pumping fluid into and out of an endoscopic surgical instrument is provided, comprising the steps of: providing a housing of a fluid pump system; inserting an inflow cartridge and an outflow cartridge into the housing; attaching a first fluid pump to the outflow cartridge; attaching a second fluid pump to the inflow cartridge; attaching the first fluid pump and the second fluid pump to the endoscopic surgical instrument; activating one or both of the first fluid pump and the second fluid pump; and regulating a fluid flow from the outflow cartridge to the endoscopic surgical instrument and fluid flow into the inflow cartridge from the endoscopic surgical instrument.

The method further includes the steps of removing the outflow cartridge from the housing and discarding the outflow cartridge. The method further includes the steps of removing the inflow cartridge from the housing and discarding the inflow cartridge. The method further includes the step of mounting the housing of the fluid pump system on the endoscopic surgical instrument. The method further includes the steps of providing at least one suction cup on the housing and placing the housing on a flat surface such that the at least one suction cup engages the flat surface.

Other devices, methods, features, and advantages of the present invention will be or become apparent to one with skill in the art upon examination of the following drawings and detailed description. It is intended that all such additional systems, methods, features, and advantages be included within this description, be within the scope of the present invention, and be protected by the accompanying claims.

### Brief Description Of The Drawings

FIG. 1 is a perspective view of a fluid pump system, and an endoscopic surgical instrument, which are not part of the invention.

FIG. 1A is a cross section view of the endoscopic surgical instrument shown in FIG. 1.

FIG. 2 is a perspective view of the fluid pump system of FIG 1.

FIG. 2A is a perspective view of fluid cartridges in accordance with an exemplary embodiment of the invention for use in the fluid pump system of Figure 1.

FIG. 3 is a top view of the fluid pump system of FIG 1.

FIG. 4 is a perspective view of a fluid pump system in accordance with the invention mounted on an endoscopic surgical instrument

FIG. 5 is a flowchart illustrating a method for pumping fluid into and out of an endoscopic surgical instrument.

### Detailed Description Of The Drawings

Exemplary embodiments of the present invention will now be described with reference to the drawings. Those of skill in the art will recognize that other configurations and arrangements of the components and features described are possible and within the scope of the present invention.

Figure 1 is a perspective view of a fluid pump system 10 for supplying fluid to and evacuating fluid from an endoscopic surgical instrument 19. Instrument 19 is an exemplary surgical instrument having an elongated shaft 20 for insertion into the body of a patient, a handle portion 21, and at least one lever 28 for operating a surgical tool that would have working elements disposed at the distal end 30 of the shaft 20. Figure 1A is a cross section of the shaft 20 taken along line IA shown in Figure 1. Figure 1A shows the various channels that run from the handle portion 21 to the distal end 30. Instrument 19 includes a working channel 31, an imaging channel 32, an illumination channel 33, an inflow channel 34, and an outflow channel 35. Instruments for use with the fluid pump system of the present invention will have at least one channel adapted to allow fluids to flow to and/or from the distal end 30. In some embodiments, only one fluid channel is necessary.

The imaging channel 32 allows for the transmission of images to the viewing opening 29 located on the handle portion 21. The viewing opening 29 may simply consist of an ocular, not unlike a normal imaging system, that allows for the viewing of a scene adjacent to the distal end 30 of the instrument 19. The viewing opening 29 may also include or link to a liquid crystal display ("LCD") screen, a lens (e.g., glass lens), a charge coupled device ("CCD") camera chip, a complementary metal oxide semiconductor ("CMOS") device, or a similar viewing apparatus that presents an image of the scene adjacent to the distal end 30 of the instrument 19. Those having ordinary skill in the art will recognize other means of providing a view of an image at the distal end 30 of the instrument 19 to the viewing opening 29 and such means will be considered to be within the scope of the present invention.

Inflow channel 34 allows for the transmission of fluids and tissue from an area located near distal end 30 up the shaft 20 towards the handle portion 21. Outflow channel 35 allows for the transmission of fluids down the shaft 20 to an area near the distal end 30 of the instrument 19.

The fluid pump system 10 includes a housing 11, which holds inflow and outflow cartridges (not shown in FIG. 1). The housing 11 includes a lid 14 on its top portion. The lid 14 has a grip portion 23 that allows for easy opening and closing by an operator of the fluid pump system 10. The lid 14 helps to retain the components of the fluid pump system 10 and to help maintain sterility in the surgical area. Although not shown in Figure 1, in some embodiments the lid 14 includes a closure device such as a snapping or clipping mechanism or the like.

The housing 11 has windows 12a and 12b on its front face 26, through which fluid levels in the inflow and outflow cartridges (not shown in FIG. 1) can be viewed. Control knobs 13a and 13b are also located on the front face 26 of housing 10. In the embodiment shown in Figure 1, control knob 13a controls the flow from the outflow cartridge by regulating the speed of an outflow pump (not shown in Figure 1). Similarly, control knob 13b controls the flow into the inflow cartridge by regulating the speed of an inflow pump (also not shown in Figure 1).

The housing 11 is generally made of a rigid material or has substantial portions thereof that are made of rigid materials. Materials that are light but strong are preferred. Many polymer types of rigid plastics can be used in accordance with the present invention. Examples of rigid plastics include low density polyethylene, high density polyethylene, polystyrene, polyvinyl chloride and polypropylene, with polyethylene terephthalate (PET). Methods of forming the housing 11, as well as other components of the fluid pump system 10, in accordance with embodiments of the present invention includes a spectrum of processes available to the plastics industry, including: extrusion and coextrusion; thermoforming; injection moldings and multi-material injection moldings, injection blow moldings; and injection-stretch blow moldings. In addition, components of the fluid pump system 10 are made of relatively inexpensive and thermoformed plastic material such as foamed and unfoamed polystyrene, ABS, PVC, PETG, polyethylene and polypropylene in other embodiments. The dimensions, such as thickness and size, of the housing 11 and other components varies depending on the particular embodiment.

As will be discussed in more detail below, the housing 11 and other components of the fluid pump system 10 may be disposable. Such components are intended to be used only once and then discarded in a sanitary fashion. By providing some or all of the components of the fluid pump system 10 as disposable, significant time and cost savings can be achieved by minimizing the number of components that must be sterilized.

On left side face 25 of housing 10 is a pump outflow port 24. Pump outflow port 24 is in fluid communication with the instrument 19 via flow tube 15. Instrument 19 has a suitable instrument inflow port 18 for connecting to and receiving fluids from flow tube 15. Right side face 27, which is not visible in Figure 1, has a pump inflow port in a suitable location on right side face 27. The pump inflow port is in fluid communication with instrument 19 via flow tube 16 and an instrument outflow port 17. Essentially, fluids from an outflow cartridge are pumped through an outflow pump out of the pump outflow port 24, through the flow tube 15, into instrument inflow port 18, and down the outflow channel 35 of the shaft 20. Fluids are thereby delivered for irrigation to an area adjacent to the distal end 30 of the instrument 19. Also, fluids and/or tissue present at an area adjacent to the distal end 30 can be transported up the inflow channel 34, out of the instrument outflow port 17, through the flow tube 16, into the pump inflow port, through the inflow pump, and finally deposited in the inflow cartridge. The inflow cartridge is preferably empty when provided in the housing 11 prior to a surgical or other procedure.

Figure 1 also shows the housing 11 having suction cups 22 on its bottom for temporarily securing it to a flat surface. The suction cups 22 are formed of a suitably flexible plastic or rubber material and are sized according to the size and weight of the housing 11 to provide adequate support. The suction cups are provided, in the embodiment shown, on each of the four bottom corners of the housing 11. In some embodiments, the suction cups 22 are removable from the housing 11 so that the fluid pump system 10 can be used in a variety of arrangements. For example, the suction cups 22 can be removed and replaced with small rubber feet which restrict sliding of the housing but allow for quick relocation during a procedure.

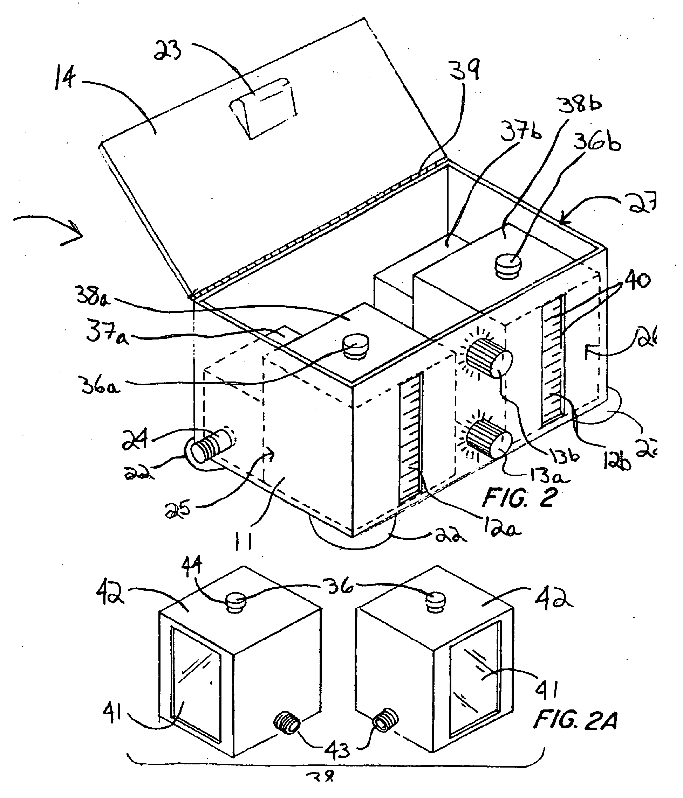

Figure 2 shows the housing 11 with the lid 14 in an open position. The lid 14 swivels on a hinge 39. More detail of the windows 12a and 12b is shown, including lines 40 that are printed on the windows. The lines 40 aid a user of the system in gauging the amount of fluid in either the outflow or inflow cartridge. The windows 12a and 12b are advantageously made of a transparent plastic or glass. In some embodiments, windows 12a and 12b are merely openings in the front face 26 of the housing 11, and do not include a separate transparent piece. In such an embodiment, lines 40 may be printed on the inflow and outflow cartridges themselves.

Control knobs 13a and 13b control the speed of the pumps 37a and 37b and thereby control the degree of fluid flow into or out of the fluid pump system 10. The knobs 13a and 13b twist so that the user can regulate the speed of the pumps from an off position to their maximum pumping speed. In other embodiments, other types of control devices are employed for regulating the pumps. For example, push buttons for activating, increasing the speed of a pump, decreasing the speed of a pump, and/or turning a pump off are used. Dials or slider controls are used in other embodiments. In the embodiment shown, pump 37a is an outflow pump and pulls fluid from the outflow cartridge 38a to be dispensed out of the pump outflow port 24. Pump 37b is an inflow pump and deposits fluid or tissue matter drawn from the pump inflow port (not shown, but substantially similar to pump outflow port 24) into the inflow cartridge 38a.

Figure 2A shows two fluid cartridges 38 for use as either an inflow or an outflow cartridge. The cartridges shown are identical, but are shown at different angles. Essentially, the cartridges 38 have a window 41 in a case 42 on both a front and a back sides and have a fluid port 43 on only one side. The window 41 is made of transparent plastic or glass and is constructed such that the window 41 and the case 42 seal the interior of the cartridge 38. Having a window on both sides of the cartridge adjacent to the side with the fluid port 43 allows for easy manufacturing since a completed fluid cartridge may be used as either an inflow or an outflow cartridge.

The fluid ports 43 are shown as threaded and include a suitable valve assembly (not shown) so that the interior of the cartridge is sealed until a flow tube connector is attached. Such a valve will be self-sealing so that fluid cannot escape the fluid port 43 once a flow tube connector is removed. A threaded surface is not necessary on the fluid port 43, but may instead be adapted to make a press-fit, snap-fit, quick connect, or other suitable connection. Of importance with regard to the fluid cartridges is that the contents of the cartridge be prevented from exposure to the external environment so that sterility of the inside of the cartridge and its contents can be maintained. Conversely, fluids and/or tissue that have been withdrawn from a patient's body must be prevented from contacting other components or tissues in order to maintain sterility.

In the embodiment shown in Figures 2 and 2A, the fluid cartridges 38 have vents 36 located on their top portions. In the embodiment shown, the vents are of a screw-type, such that when a user of the system wishes to provide venting, he or she simply unscrews the top portion 44 of the vent a selected amount. In other embodiments, the vent is located on other portions of the fluid cartridge and other types of vents are used. In some embodiments, the vent may be suitable for use by the manufacturer or user in filling the cartridge with fluid for outflow.

Fluid cartridges 38 are sufficiently simple and inexpensive to manufacture that they are provided as single-use, disposable units. When a fluid cartridge 38 is provided as an outflow cartridge, it will be pre-filled by the manufacturer with any one of the many fluids that are useful in endoscopic surgical techniques. For example, laparoscopic, arthroscopic and hysteroscopic procedures require sufficient volumes of irrigation fluid to be delivered to the surgical site in order to maintain tamponade, isolate bleeders and to generally clear the surgical area. Therapeutic maneuvers during hysteroscopy, for example, include taking a tissue sample, known as a biopsy, removal of polyps or fibroid tumors, or preventing bleeding with cautery; freezing, heat or chemicals, during a hysteroscopy procedure that may require irrigation fluid. The irrigation fluid may encompass any fluid that may be delivered into a body cavity during a medical procedure, and, for example, may include gases or solid elements mixed into a fluid solution. Fluids and solutions such as saline, glycine, lactated Ringer's solution, or the like and various other compositions that may be delivered fluidly into a patient, are useful in a variety of surgical techniques and may be supplied in a disposable fluid cartridge 38 for use as an outflow cartridge. On the other hand, when a fluid cartridge 38 is provided as an inflow cartridge, it is provided empty. The inflow cartridge is simply installed in the housing 11, attached to the inflow pump 37b, and vented if necessary.

While not shown in the Figures, the housing 11 and the fluid cartridges 38 include means for securing the cartridges 38 in the housing 11. In embodiments of the present invention, securing means such as clips or snaps provided on the case 42 of the cartridge 38 and/or the inside surfaces of the housing 11 are provided for securing the cartridges 38 and preventing them from sliding or tipping inside the housing 11.

The fluid cartridges 38 are constructed in varying sizes in various embodiments of the present invention. In some embodiments, the fluid cartridges 38 used as for inflow cartridges and for outflow cartridges may be configured to hold about 200cc of fluid, about 150cc of fluid, about 100cc of fluid, or about 50cc of fluid, depending on the overall size of the fluid pump system 10 being utilized and the requirements of the surgeon. In yet other embodiments, the inflow cartridge and the outflow cartridge may be larger. The inflow cartridge and the outflow cartridge may hold about 300cc of fluid, about 350cc of fluid, about 400cc of fluid, about 500cc of fluid, or more without departing from the scope of the present invention. Further, while in the embodiment shown in Figures 1-3 the inflow cartridge 38b and the outflow cartridge 38a are of the same size, the inflow cartridge and the outflow cartridge may have different sizes in other embodiments.

The fluid pump system 10 is designed so that the inflow and outflow cartridges can be quickly and easily installed and removed in the system. This facilitates swapping fluid cartridges during a surgical or other procedure if the need to do so arises. For example, some surgical procedures may require more than one type of irrigation fluid or solution, or may require so much irrigation fluid or solution that more than one full cartridge is necessary. Also, some surgical procedures may require evacuation of a volume of fluid or tissue that exceeds a single inflow cartridge. In such cases, a user of the system can simply remove the empty outflow cartridge or full inflow cartridge and replace it so that the surgical procedure can continue.

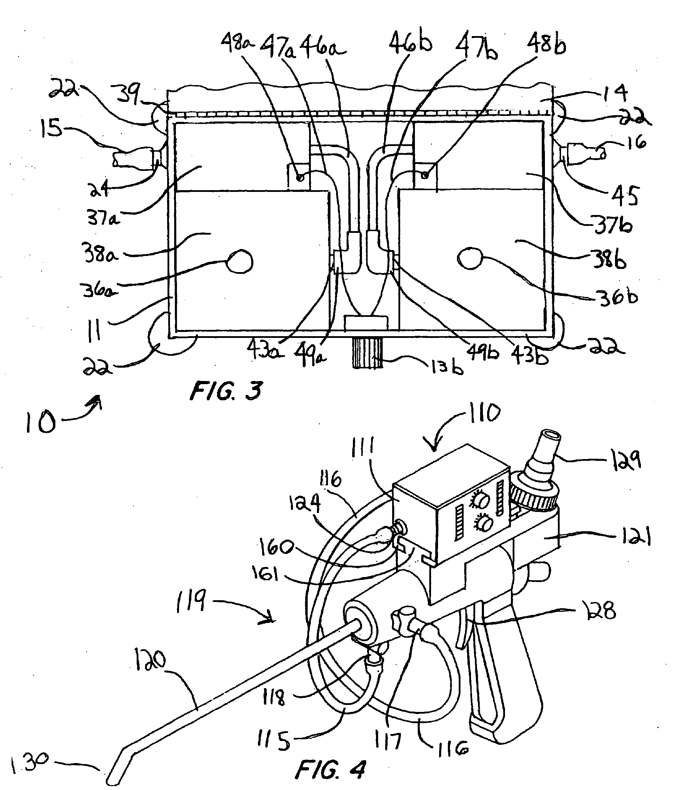

Figure 3 is a top view of the fluid pump system 10 shown in Figures 1 and 2. The spatial relationship between the inflow cartridge 38b, the inflow pump 37b, the outflow cartridge 38a, and the outflow pump 37a within the housing 11 is visible. Pump inflow port 45 is shown in a location that corresponds to the location of pump outflow port 24. Also visible in Figure 3 are the wires 47a and 47b which connect the control knobs 13a and 13b with the outflow pump 37a and the inflow pump 37b, respectively. The wires 47a and 47b are connected at connection points 48a and 48b to the pumps 37a and 37b.

The fluid connections between the pumps and the cartridges are also shown in Figure 3. Tubes 46a and 46b attach to the fluid ports 43a and 43b on the sides of the outflow and inflow cartridges by connectors 49a and 49b. The tubes 46a and 46b carry fluids to/from the pumps 37a and 37b.

The pumps 37a and 37b utilized in fluid pump systems according to the present invention may be one of any number of types of pumps that are known to those of skill in the art. The pumps may be, for instance, one or more dynamic pumps or positive displacement pumps in accordance with the fourth exemplary embodiment. Dynamic pumps include, but are not limited to: centrifugal pumps and axial pumps. Positive displacement pumps include, but are not limited to: reciprocating pumps, metering pumps, rotary pumps, peristaltic pumps, and moyno pumps. The type of pump selected depends on the size, intended application, and other factors unique to the particular embodiment in question.

The pumps 37a and 37b, like the fluid cartridges 38, are disposable in some embodiments of the present invention. For some pump types, providing them as disposable units is more economical than requiring them to be sterilized after every use. For other pump types, it may be more economical to sterilize them due to the cost of their manufacture. In some embodiments, some components of the pumps are disposable while others are retained and reused. For example, in a peristaltic pump, only the flexible tubing is potentially exposed to non-sterile material, while the working parts are not compromised during operation. In such a case, only the flexible tubing need be disposed of and replaced, while the remainder of the pump, and presumably the most costly components of the pump, can be retained and reused. Such an arrangement is also possible with other types of pumps in addition to the peristaltic type.

In some embodiments, the pumps are driven by small electric motors that are contained within the casing of the pump. These electric motors may be powered by a small internal battery or by an external power source. Such an external power source may be mounted on the outside of the housing 11 of the fluid pump system 10 and may derive power from a battery or electric outlet. The pumps 37a and 37b are secured within the housing 11 by suitable means, such as clips or snaps, to prevent the pumps from moving and potentially becoming disconnected from the fluid cartridges 38 and/or the control knobs 13. In other embodiments, the pumps are mounted on the outside of the housing 11 by suitable clips. In such arrangements, the pumps interact with the other components of the system 10 via openings or other connections in the back of the housing 11.

It will be understood by those of skill in the art that fluid pump systems according to the present invention need not include both the provision for the inflow of fluids (inflow cartridge and inflow pump) and for the outflow of fluids (outflow cartridge and outflow pump). A surgeon may know, prior to surgery, that he or she will need only to irrigate the surgical site, or that he or she will need only to evacuate fluids and tissue from the surgical site. In such a case, the fluid pump system may be provided with only those components that are necessary.

Figure 4 is a perspective view of the present invention. In this embodiment, a fluid pump system 110 is mounted directly on the handle portion 121 of an endoscopic surgical instrument 119. The details of the instrument 119 are similar to those of instrument 19 shown in Figure 1. The instrument 119 has a shaft 120, having channels running along its length, a handle portion 121, at least one lever 128, a distal end 130, and a viewing opening 129.

In the embodiment shown in Figure 4, however, the instrument 119 includes a mounting area 161 which securingly receives an appropriately designed fluid pump system 110. The fluid pump system 110 communicates with the outflow and inflow channels of the instrument 119 via flow tubes 115 and 116 and instrument inlet port 118 and instrument outlet port 117. The housing 111 of fluid pump system 110 has securing means 160 formed on its bottom surface for mating with the mounting area 161 of the instrument 119. In the embodiment shown, the fluid pump system 110 is mounted on the instrument 119 using a tongue and groove type design. The system 110 is retained on the mounting area 161 despite movements of the instrument 119 by for example, friction due to a snug fit, snaps, clips, or the like. One of skill in the art will appreciate that other means of securing the fluid pump system 110 to the instrument 119 are within the scope of the present invention. For example, plastic clips, hinged clips, spring-release pegs and recesses, and the like.

The embodiment shown in Figure 4 is particularly advantageous because it can provide the most convenient access to the fluid pump system 110 for the surgeon or operator of the tool 119. Whenever the surgeon desires to produce or regulate the flow of fluid, he or she need not reach or remove his or her hands from the instrument for very long. In this embodiment, the fluid pump system 110 is most advantageously a small size, and therefore the outflow and inflow cartridges are also small and have a limited fluid capacity. Small size ensures that the fluid pump system 110 does not impede the surgeon in performing surgical tasks by being in the way or making the instrument 119 too heavy or cumbersome.

Further, the fluid pump system 110 is, in some embodiments, also adapted to utilize suction cups or other "feet" so the system can be effectively used on a flat surface, instead of mounted directly on the instrument 119. As stated above, these suction cups or other "feet" are removable and would be removed whenever the system was to be mounted on an instrument 119. Thus, fluid pump system 110 provides a versatile solution to the fluid supply and evacuation needs of surgeons.

With regard to the disposability of the system, in some embodiments, all of the components, including the housing are intended to be used only once and then discarded. In other embodiments, the fluid cartridges, pumps, flow tubes, and connectors are disposable, while the housing is to be retained and used with replacement components. In still other embodiments, only the fluid cartridges and some parts of the pumps are disposable, while the remaining components of the system are reusable. One of skill in the art will appreciate that many combinations of disposable and reusable components are possible and within the scope of the present invention.

Figure 5 is a flowchart illustrating a method for pumping fluid into and out of an endoscopic surgical instrument. First, a housing of a fluid pump system is provided at 200. Next, an inflow cartridge and an outflow cartridge are inserted into the housing at 201. Then, a first fluid pump is attached to the outflow cartridge such that fluid communication is possible between them at 202. Then, a second fluid pump is attached to the inflow cartridge such that fluid communication is possible between the second fluid pump and the inflow cartridge at 203. Next, the first fluid pump and the second fluid pump are attached to the endoscopic surgical instrument such that they are in fluid communication with the endoscopic surgical instrument at 204. Then, one or both of the first fluid pump and the second fluid pump are activated at 205 and a fluid flow from the outflow cartridge to the endoscopic surgical instrument and fluid flow into the inflow cartridge from the endoscopic surgical instrument are regulated at 206.

Figure 5 shows blocks 209, 210, and 211 that are incorporated in some cases of the methods according to the present invention. In some cases, the housing of the fluid pump system is mounted on the endoscopic surgical instrument directly for the convenience of the surgeon at 209. It will be understood by those of ordinary skill in the art that, while this step or steps are shown in the Figure as optionally taking place between blocks 200 and 201, it may be performed at other appropriate points in the method. Similarly, in other cases, at least one suction cup is provided on the housing at 210 and the housing is placed on a flat surface such that the at least one suction cup engages the flat surface at 211. These additional steps may optionally be performed at various, other appropriate points in the method.

Finally, 207 and 208 include the steps of removing the outflow cartridge from the housing and discarding it and the steps of removing the inflow cartridge from the housing and discarding it, respectively. These steps most often will take place after the conclusion of a surgery, but, as mentioned above, in surgeries where multiple outflow cartridges or inflow cartridges are needed these steps may take place at other points in the method.

It should be noted that any process descriptions or blocks in flow charts should be understood as representing modules, segments, portions of code, or steps that include one or more instructions for implementing specific logical functions in the process; in alternate implementations functions may be executed out of order from that shown or discussed, including substantially concurrently or in reverse order, depending on the functionality involved, as would be understood by those reasonably skilled in the art of the present invention.

Thus, the present invention provides a fluid pump system for an endoscopic surgical instrument that is simple and easy to maintain and use, inexpensive to maintain and manufacture, lightweight and unobtrusive for use in a variety of settings, and extremely versatile.

It should be emphasized that the above-described embodiments of the present invention, particularly, any "preferred" embodiments, are merely possible examples of implementations, merely set forth for a clear understanding of the principles of the invention. Many variations and modifications may be made to the above-described embodiments of the invention without departing substantially from the principles of the invention. All such modifications and variations are intended to be included herein within the scope of this disclosure and the present invention and protected by the following claims.

## Claims

1. A fluid pump system for an endoscopic surgical instrument (19, 119), the system (10, 110) comprising:
a housing (11, 111);
a disposable inflow cartridge (38b), detachably connected to the housing (11, 111); a disposable outflow cartridge (38a), detachably connected to the housing (11, 111); and at least one fluid pump (37a, 37b) connected to the housing (11, 111);
wherein the at least one fluid pump (37a, 37b) is in communication with at least one of the disposable inflow cartridge (38b) and the disposable outflow cartridge (38a), **characterized by** an endoscopic surgical instrument (19, 119) having a shaft (20, 120) for insertion into the body of a patient, and in that the housing (111) is detachably mounted to the endoscopic surgical instrument (119).

2. The fluid pump system of claim 1, wherein the endoscopic surgical instrument (19, 119) is in fluid communication with at least one of the disposable inflow cartridge (38b) and the disposable outflow cartridge (38a).

3. The fluid pump system of claim 1, wherein the housing (11, 111) includes at least one control device (13a) for controlling the at least one fluid pump (37a), thereby regulating the flow of an irrigation fluid from the outflow cartridge (38a).

4. The fluid pump system of claim 1, wherein the housing (11, 111) includes at least one control device (13b) for controlling the at least one fluid pump (37b), thereby regulating the flow of an irrigation fluid into the inflow cartridge (38b).

5. The fluid pump system of claim 1, wherein the housing (11) includes at least one suction device (22) for securing the housing (11) on a surface.

6. The fluid pump system of claim 1, wherein the at least one fluid pump (37a, 37b) is detachably connected to the housing (11, 111) and is disposable.

7. The fluid pump system of claim 1, wherein a first fluid pump (37a) is in fluid communication with the inflow cartridge (38b) and a second fluid pump (37b) is in fluid communication with the outflow cartridge (38a).

8. The fluid pump system of claim 7, wherein the first fluid pump (37a) is in fluid communication with an inflow channel (34) of the endoscopic surgical instrument (19, 119) and the second fluid pump (37b) is in fluid communication with an outflow channel (35) of the endoscopic surgical instrument (19, 119).

9. The fluid pump system of claim 1, wherein the first pump (37a) and the second pump (37b) are detachable connected to the housing (11, 111)

10. The fluid pump system of claim 1, wherein said housing (111) has securing means (160) formed on its bottom surface for mating with a mounting area (161) of the instrument (119).

11. The fluid pump system of claim 10, wherein the housing (111) is retained on the mounting area (161) by friction, due to a snug fit, snaps, clips, plastic clips, hinged clips, spring-release pegs or recesses.

## Patentansprüche

1. Fluidpumpensystem für ein endoskopisches Operationsinstrument (19, 119) wobei das System (10, 110) folgendes aufweist, nämlich
- ein Gehäuse (11, 111);
- eine wegwerfbare Zuflusskartusche (38b), die lösbar mit dem Gehäuse (11, 111) verbunden ist;
- eine wegwerfbare Abflusskartusche 38a, die lösbar mit dem Gehäuse (11, 111) verbunden ist;
- und zumindest eine Fluidpumpe (37a, 37b), die mit dem Gehäuse (11, 111) verbunden ist;
- wobei die zumindest eine Fluidpumpe (37a, 37b) zumindest mit einer der wegwerfbaren Zuflusskartusche (38b) oder der wegwerfbaren Abflusskartusche (38a) in Verbindung steht, **dadurch gekennzeichnet, dass** das endoskopische Operationsinstrument (19, 119) einen Schaft (20, 120) zum Einführen in den Körper eines Patienten aufweist, und dass das Gehäuse (111) lösbar an dem endoskopischen Operationsinstrument (119) angebracht ist.

2. Fluidpumpensystem nach Anspruch 1, wobei das endoskopische Operationsinstrument (19, 119) in strömungstechnischer Verbindung mit zumindest einer der wegwerfbaren Zuflusskartusche (38a) oder der wegwerfbaren Abflusskartusche (38a) steht.

3. Fluidpumpensystem nach Anspruch 1, wobei das Gehäuse (11, 111) zumindest eine Steuervorrichtung (19a) zum Steuern der zumindest einen Fluidpumpe (37a) aufweist, wodurch der Fluss eines Spülfluids aus der Zuflusskartusche (38a) regelbar ist.

4. Fluidpumpensystem nach Anspruch 1, wobei das Gehäuse (11, 111) zumindest eine Steuervorrichtung (13b) zum Steuern der zumindest einen Fluidpumpe (37b) aufweist, wodurch der Fluss an Spüffluid in die Zuflusskartusche (38b) regulierbar ist.

5. Fluidpumpensystem nach Anspruch 1, wobei das Gehäuse (11) zumindest eine Saugvorrichtung (22) aufweist, um das Gehäuse (11) an einer Oberfläche zu befestigen.

6. Fluidpumpensystem nach Anspruch 1, wobei die zumindest eine Fluidpumpe (37a, 37b) lösbar an dem Gehäuse (11, 111) befestigt ist und das diese wegwerfbar ist.

7. Fluidpumpensystem nach Anspruch 1, wobei eine erste Fluidpumpe (37a) strömungstechnisch mit der Zuflusskartusche (38b) und eine zweite Fluidpumpe (37b) strömungstechnisch mit der Abflusskartusche (38a) in Verbindung steht.

8. Fluidpumpensystem nach Anspruch 7, wobei die erste Fluidpumpe (37a) in strömungstechnischer Verbindung mit einem Zuflusskanal (34) des endoskopischen Operationsinstrumentes (19, 119) steht, und die zweite Fluidpumpe (37b) in strömungstechnischer Verbindung mit einem Abflusskanal (35) des endoskopischen Operationsinstrumentes (19, 119) steht.

9. Fluidpumpensystem nach Anspruch 1, wobei die erste Pumpe (37a) und die zweite Pumpe (37b) lösbar am Gehäuse (11, 111) befestigt sind.

10. Fluidpumpensystem nach Anspruch 1, wobei das Gehäuse (111) an seiner Bodenfläche Montagemittel (160) aufweist, um mit einem Montagebereich (161) des Instruments (119) passend zusammenfügbar zu sein.

11. Fluidpumpensystem nach Anspruch 10, wobei das Gehäuse (111) an dem Montagebereich (116) durch Reibung, Passsitz, Schnappverbindungen, Clips, Kunststoffclips, gelenkige Clips, federbelastete Bolzen oder Aussparungen gehalten ist.

## Revendications

1. Système de pompe à fluide destiné à un instrument chirurgical endoscopique (19, 119), le système (10, 110) comprenant :
un logement (11, 111) ;
une cartouche d'écoulement entrant jetable (38b), connectée de manière amovible au logement (11, 111) ; une cartouche d'écoulement sortant jetable (38a), connectée de manière amovible au logement (11, 111) ; et au moins une pompe à fluide (37a, 37b) connectée au logement (11, 111) ;
dans lequel la au moins une pompe à fluide (37a, 37b) est en communication avec au moins une cartouche parmi la cartouche d'écoulement entrant jetable (38b) et la cartouche d'écoulement sortant jetable (38a), **caractérisé par** un instrument chirurgical endoscopique (19, 119) présentant un axe (20, 120) destiné à une insertion dans le corps d'un patient, et en ce que le logement (111) est monté de manière amovible sur l'instrument chirurgical endoscopique (119).

2. Système de pompe à fluide selon la revendication 1, dans lequel l'instrument chirurgical endoscopique (19, 119) est en communication de fluide avec au moins une cartouche parmi la cartouche d'écoulement entrant jetable (38b) et la cartouche d'écoulement sortant jetable (38a).

3. Système de pompe à fluide selon la revendication 1, dans lequel le logement (11, 111) inclut au moins un dispositif de commande (13a) destiné à commander la au moins une pompe à fluide (37a), régulant ainsi l'écoulement d'un fluide d'irrigation provenant de la cartouche d'écoulement sortant (38a).

4. Système de pompe à fluide selon la revendication 1, dans lequel le logement (11, 111) inclut au moins un dispositif de commande (13b) destiné à commander la au moins une pompe à fluide (37b), régulant ainsi l'écoulement d'un fluide d'irrigation dans la cartouche d'écoulement entrant (38b).

5. Système de pompe à fluide selon la revendication 1, dans lequel le logement (11) inclut au moins un dispositif d'aspiration (22) destiné à fixer le logement (11) sur une surface.

6. Système de pompe à fluide selon la revendication 1, dans lequel la au moins une pompe à fluide (37a, 37b) est connectée de manière amovible au logement (11, 111) et est jetable.

7. Système de pompe à fluide selon la revendication 1, dans lequel une première pompe à fluide (37a) est en communication de fluide avec la cartouche d'écoulement entrant (38b) et une deuxième pompe à fluide (37b) est en communication de fluide avec la cartouche d'écoulement sortant (38a).

8. Système de pompe à fluide selon la revendication 7, dans lequel la première pompe à fluide (37a) est en communication de fluide avec un canal d'écoulement entrant (34) de l'instrument chirurgical endoscopique (19, 119) et la deuxième pompe à fluide (37b) est en communication de fluide avec un canal d'écoulement sortant (35) de l'instrument chirurgical endoscopique (19, 119).

9. Système de pompe à fluide selon la revendication 1, dans lequel la première pompe (37a) et la deuxième pompe (37b) sont connectées de manière amovible au logement (11, 111).

10. Système de pompe à fluide selon la revendication 1, dans lequel ledit logement (111) présente un moyen de fixation (160) formé sur sa surface inférieure destiné à correspondre à une zone de montage (161) de l'instrument (119).

11. Système de pompe à fluide selon la revendication 10, dans lequel le logement (11) est retenu sur la zone de montage (161) par friction, en raison d'un ajustement à frottement doux, de dispositifs d'emboîtement, de clips, de clips en plastique, de clips à charnières, de pinces à ressort ou d'évidements.
